(19) **Europäisches Patentamt**

**European Patent Office**

**Office européen des brevets**

(11) **EP 0 846 441 B1**

(12) **EUROPEAN PATENT SPECIFICATION**

(45) Date of publication and mention
of the grant of the patent:
**26.05.2004 Bulletin 2004/22**

(51) Int Cl.7: **A61B 5/103**, G01L 1/20

(21) Application number: **97121153.7**

(22) Date of filing: **02.12.1997**

(54) **Apparatus for telemetering interaction forces between the foot and the ground in a subject walking**

Gerät zur Fernübertragung von Wechselwirkungskräften zwischen dem Fuss und der Erde von einer gehenden Person

Appareil de télétransmission des forces d'interaction agissant entre le pied et le sol durant la marche

(84) Designated Contracting States:
**AT BE CH DE DK FR GB IT LI NL SE**

(30) Priority: **04.12.1996 IT RM960834**

(43) Date of publication of application:
**10.06.1998 Bulletin 1998/24**

(73) Proprietor: **ISTITUTO SUPERIORE DI SANITA'**
**00161 Roma (IT)**

(72) Inventors:
 • **Macellari, Velio**
  **00125 Roma (IT)**
 • **Fadda, Antonello**
  **00182 Roma (IT)**

(74) Representative: **Gervasi, Gemma, Dr.**
**Notarbartolo & Gervasi S.p.A.,**
**Corso di Porta Vittoria, 9**
**20122 Milano (IT)**

(56) References cited:
 EP-A- 0 172 784         WO-A-87/01574
 US-A- 5 323 650

 • C. ALVARADO ET AL: "TELEMETRY SYSTEM FOR CONTINUOUS MEASUREMENT OF VERTICAL FOOT FORCES DURING WALKING" PROCEEDINGS OF THE INSTRUMENTATION AND MEASUREMENT TECHNOLOGY CONFERENCE, WALTHAM, MA., APR. 23 - 26, 1995, 23 April 1995, INSTITUTE OF ELECTRICAL AND ELECTRONICS ENGINEERS, pages 581-583, XP000534919

**Description**

State of the art.

Both at a research level and at a commercial one various sensitive insoles have been proposed, which comprise a number of sensors and must be inserted in a shoe for measuring the distribution of the forces under the foot.
Such sensors are sensitive only to the vertical component of the force and, consequently, since the area taken by the sensors is known, they actually measure the pressure locally exchanged between the foot and the ground, and are therefore usually referred to as pressure sensors.
Sensitive insoles always consist of a discrete set of sensors, whose number may vary from a few sensors (e. g., 16) positioned at points corresponding to characteristic points of the foot sole up to as many as a thousand of sensors positioned and arranged according to a matrix.
The most usually used technology (because it is the least expensive one) produces sensors of resistive type in which the electric resistance is a decreasing function of the pressure; just to provide examples, the following may be cited:

- a sensitive insole with sensors spaced 5 mm apart (produced by the firm Tekscan Inc.), which is sold as an accessory (with a life limited to a few dozen measurements) of a complete measuring system which involves a cable connection with the subject;
- a sensitive insole (produced by the firm Interlink, involved only in the sale of sensors) consisting of a mylar film where 64 sensors are laid out, arranged according to a not altogether regular matrix.

Finally, a sensor produced by the firm Interlink and proposed for realising push-button panels of special type is cited, which is suitable for measuring the magnitude and the position of a force in a plane. As expressly stated by the firm Interlink itself, this sensor cannot, however, be adapted and/or used for the production of sensitive insoles to be inserted in shoes.
One form of presentation of the information of these devices consists simply of the image of the foot sole with superimposed thereon the responses of the individual sensors coded according to an arbitrary colours scale.
It is possible to show both the successive instantaneous responses recorded at regular time intervals (with a lower limit of about 10 msec) and the maximum values measured by the individual transducers for the entire period in which the foot is pressing down. In any case, this type of presentation, which is a qualitative one, must be combined with numerical information if more precise evaluations are needed, such as those necessary for comparing results of different kinds of therapy and for following the effects thereof over time.
A very effective synthesis of this type of information is obtained by representing on the foot sole the succession of the points of application of the resultant force of reaction of the entire foot associated, if possible, to the values of the vertical component of said force: due to the slight inclination of the resultant force of reaction with respect to the vertical, said values practically coincide with those of the resultant force of reaction. This result is obtained by processing (at each sampling instant) the responses of all the sensors taking into account their position in the matrix. This operation calls for a certain computation power and is therefore normally carried out off-line at the end of the data acquisition step.
The sensitive insoles currently available allow the measurement of the distribution of the forces under the foot sole during the entire phase in which the foot is pressing against the ground, but the acquisition of the responses of all the sensors is, however, not compatible with the limited capacity of a telemetering channel, particularly if the aim is to keep costs down. It is therefore advantageous to pre-process the information to be transmitted by means of an electronic circuit which, once it has received the responses of all the sensors of the sensitive insole, supplies instant by instant a limited amount of information to be transmitted to the microprocessor.
US 5,323,650 discloses a system comprising a force sensor array and an electronic circuit module for scanning the sensor array to obtain a measurement of the force sensed by each of the sensors.
In the device to which the present invention refers, a pre-processing unit calculates the vertical component of the resultant of the forces of interaction between the foot and the ground, thus allowing an on-line monitoring via radiotelemetering.
In this case, in fact, only three quantities need to be transmitted for each foot (the vertical component of the resultant force and the co-ordinates of its point of application) and a low-capacity and low-cost transmission channel is therefore suitable for this purpose.
In fact, a transmission rate of the transmission channel of about 4800 bits/sec with a sampling frequency of 100 Hz and an 8-bit analog-to-digital converter are adequate.
The acquisition of the responses of all the sensors would instead involve a data flow that would prove hard to handle for a radiotelemetering channel unless the number of sensors is very low.
As an alternative to radiotelemetering, a cable connection or the storage on a local storage means would limit the duration of the observation times to a few minutes.

The displacement of the centre of pressure during the time in which the foot is exerting pressure is sufficiently representative of the complexity of the foot-ground interaction and of its possible pathological alteration.

Summary of invention

The present invention refers to an apparatus for measuring the vertical component of the resultant force of the foot-ground interaction and of the co-ordinates (in a reference system integral with the foot) of its point of application, comprising at least one sensitive insole (to be inserted in a shoe) equipped with a number of force sensors of resistive type and a pre-processing unit associated to the at least one sensitive insole.

The force sensors of resistive type are arranged in a matrix addressable by rows and columns, where one end of each row, respectively of each column of the matrix is connected to a generator supplying a voltage whose intensity is proportional to the co-ordinate of the row, respectively of the column in the aforesaid reference system, and the co-ordinates of the point of application of the vertical component of the resultant force are correlated to the voltage measured at the other ends of the rows, respectively of the columns, connected together in parallel. The vertical component of the resultant force is correlated to the intensity of the current supplied by a voltage generator connected to one end of the rows (or of the columns) of the matrix, connected together in parallel, when the other ends of the rows (or of the columns) of the matrix are connected to ground.

The pre-processing unit comprises at least one logical unit and switching means activated by the logical unit and suitable for connecting the voltage generators to one end of the rows, respectively of the columns, and for connecting the other ends of the rows, respectively of the columns of the matrix to a scanning unit (in turn connected to the logical unit via an analog-to-digital converter), respectively to ground.

List of figures

The invention will now be described in greater detail with reference to a nonlimiting embodiment shown in the attached figures, where:

- Figure 1 shows a schematic representation of an apparatus according to the invention;
- Figures from 2 to 5 show schematic circuit diagrams and a graph suitable for illustrating the working of an apparatus according to the invention;
- Figure 6 shows a schematic representation of the equivalent circuit of a sensitive insole S and a block diagram of the pre-processing unit P associated to it;
- Figure 7 shows a simplified flowchart to better illustrate the working of the logical unit 4 of Figure 6.

Detailed description

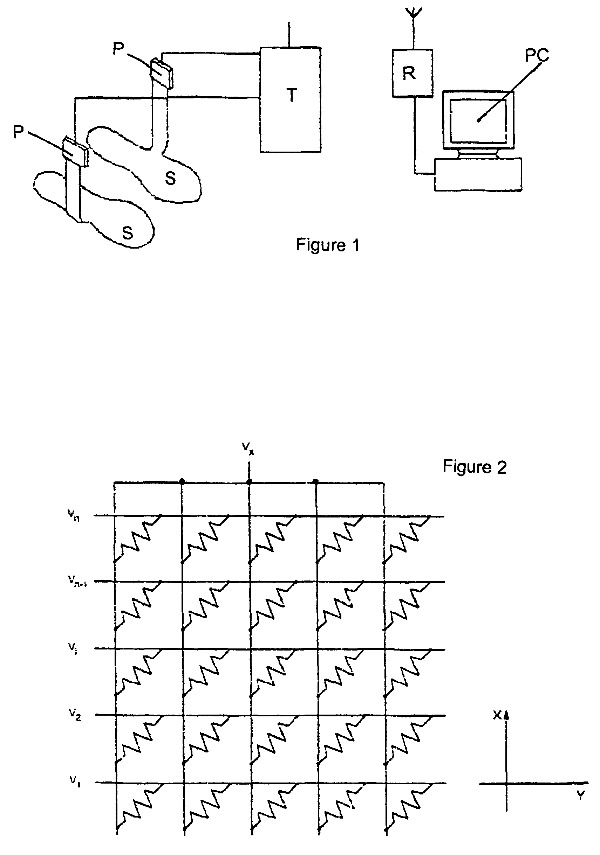

Figure 1 shows a schematic representation of an apparatus according to the invention. In Figure 1 there are shown two sensitive insoles S, the pre-processing units P associated to each sensitive insole S and the transceiver apparatus (T, R) which connects via radio the pre-processing units P to the processing unit PC, if present.

The sensitive insoles S and the associated pre-processing units P will be described in greater detail with reference to the following figures. The transceiver apparatus (T, R) and the processing unit PC (which could be omitted if no further processing of the data pre-processed by the units P is required) will not be described herein since they are known and anyway outside the present invention.

Figure 2 shows a schematic circuit diagram used for calculating a co-ordinate (conventionally the vertical one, x) of the point of application of the resultant force.

The sensitive insole S is represented by a number of resistors, whose resistance is a function of the local pressure, arranged in a matrix addressable by rows and columns.

Each row of the matrix is supplied by a generator supplying a voltage $V_i$ that is proportional to the co-ordinate x of the row in a reference system integral with the foot. In a preferred embodiment, these voltage generators $V_i$ are realised by means of a voltage generator connected to a resistance divider consisting of resistors whose values are sufficiently low that the equivalent impedance of the generators is negligible with respect to the minimum value of resistance resulting from the parallel of all the sensors connected to one row, assuming that they are simultaneously subject to the maximum pressure.

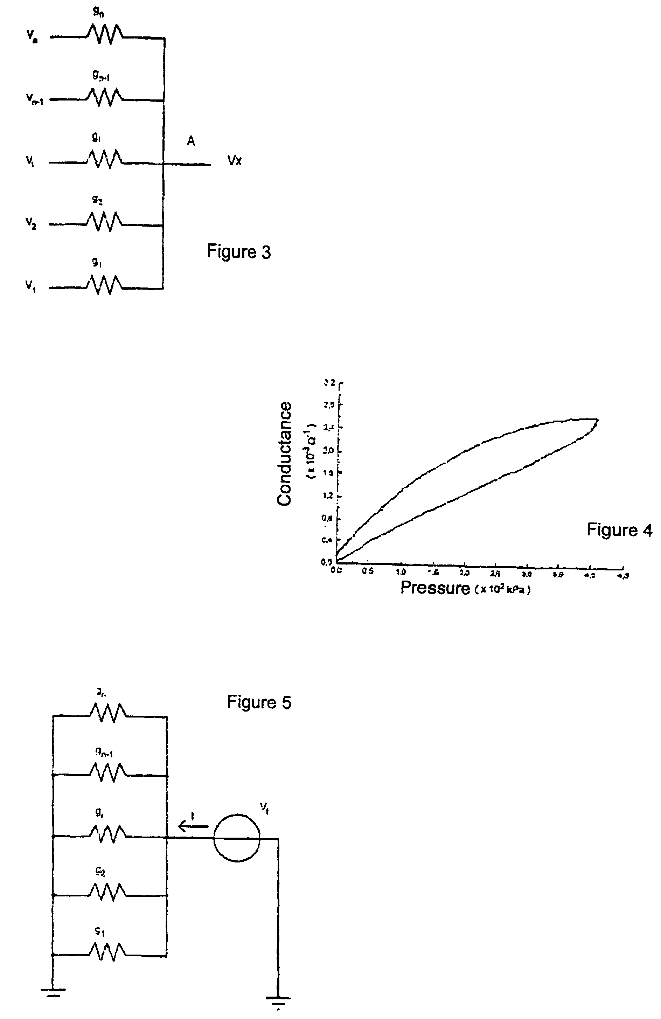

Figure 3 shows the equivalent circuit of the diagram of Figure 2, where the conductance $g_i$ is the sum of the conductance of the sensors belonging to the i-th row of the matrix.

The output voltage $V_x$ can be calculated as follows:

Given:

1) $g_i = \beta \cdot F_i$ (the conductance of the i-th row is proportional to the force acting on the entire row),
2) $V_I = \alpha \cdot x_i$ (the voltage applied to the i-th row is proportional to its co-ordinate in the above-mentioned reference system),
by imposing the equilibrium of the currents at the node A we obtain
3)

$$\sum_{i=1}^{n}(V_i - V_x) \cdot g_i = 0 \qquad V_x = \frac{\sum_{i=1}^{n} V_i \cdot g_i}{\sum_{i=1}^{n} g_i} \ ,$$

whence, by substituting Equations (1) and (2) in Equations (3), we obtain
4)

$$V_x = \frac{\sum_{i=1}^{n} \alpha \cdot x_i \cdot \beta \cdot F_i}{\sum_{i=1}^{n} \beta \cdot F_i} = \alpha \cdot x_b$$

From the above formula it is clear that the co-ordinate $x_b$ of the point of application of the vertical component of the resultant force is correlated to the voltage $V_x$; in particular, if the conductance of the sensors is linearly proportional to the pressures and therefore to the vertical components of the forces acting on the sensors, the co-ordinate $x_b$ of the point of application of the vertical component of the resultant force is proportional to the voltage $V_x$.

If the above procedure is repeated exchanging the rows of the matrix of sensors with the columns of said matrix, a voltage $V_y$ representing the co-ordinate $y_b$ of the point of application of the vertical component of the resultant force is calculated.

Figure 4 shows a typical response of a resistive pressure sensor to a pressure cycle whose amplitude and duration are similar to those that may be encountered during walking. It is immediately found that, within the precision limits of these sensors (which are usually affected by large non-linearity and hysteresis), their conductance approximates a linear behaviour, especially in the ascending part of the cycle.

It may then be concluded that the voltages $V_x$ and $V_y$ represent, with an approximation which is acceptable for the specific application, the co-ordinates of the point of application of the vertical component of the resultant force.

A source of error comes from the presence of static loads (which are different from zero even in the resting situation) due mainly to the deformation stresses that the sensitive insole S undergoes as a result of its insertion in the shoe, or to the appearance of a known term in the relation that links the conductance of a sensor to the force acting on the sensor.

In this case, the conductance of the i-th row is $g_i = \beta \cdot F_i + g_{i0}$.

Given the particular working of the sensitive insole S, said local stresses at rest (and/or said known term) give rise to an overall effect that can be represented by a pre-loading force $F_0$ applied in a point of co-ordinates $(x_0, y_0)$: this is equivalent to a linear transformation of the reference system, which can be easily amended by applying the inverse transformation.

Combining the pre-loading force $F_0$ with the force $F_b$ to be measured, we obtain
5)

$$x_r = \frac{F_b \cdot x_b + F_0 \cdot x_0}{F_b + F_0}$$

where $x_r$ is the co-ordinate of application of the resultant force, and $x_b$ is the co-ordinate of the point of application of the force $F_b$ ; the resultant force $F_r$ is
6) $F_r = F_b + F_0$.

Deriving $F_b$ from Equation (6) and substituting in Equation (5), we obtain

$$x_r = \frac{(F_r - F_0) \cdot x_b + F_0 \cdot x_0}{F_r} = x_b \cdot \left(1 - \frac{F_0}{F_r}\right) + x_0 \cdot \frac{F_0}{F_r}$$

whence

$$x_b = \frac{x_r - x_0 \cdot \dfrac{F_0}{F_r}}{1 - \dfrac{F_0}{F_r}};$$

Applying the translation of co-ordinates $X = x - x_0$ we have

$$X_b + x_0 = \frac{X_r + x_0 - x_0 \cdot \dfrac{F_0}{F_r}}{1 - \dfrac{F_0}{F_r}} = \frac{X_r + x_0 \cdot \left(1 - \dfrac{F_0}{F_r}\right)}{1 - \dfrac{F_0}{F_r}} = \frac{X_r}{1 - \dfrac{F_0}{F_r}} + x_0$$

whence, given

$$\lambda = \frac{1}{1 - \dfrac{F_0}{F_r}} = 1 + \frac{F_0}{F_b},$$

finally we obtain
7)

$$X_b = \lambda \cdot X_r$$

If we set the origin of the reference system in the point $(x_O, y_O)$ where the pre-loading force $F_O$ is applied, the co-ordinate $x_b$ of the point of application of the force $F_b$ is obtained simply by applying the corrective factor $\lambda$ to the measured value.

It is moreover necessary to pay attention to the fact that the factor $\lambda$ diverges for $F_b \ll F_0$: in other words, it may be said that, for small forces, $X_r$ is strongly attracted by the origin $x_O$, and hence there is a large corrective factor required to re-establish the correct position.

In order to prevent an amplification at the same time of the inevitable errors of measurement and of quantization included in $X_r$, it is necessary to limit the application of Equation (7) to the cases where $F_b \geq F_O$, which are markedly prevalent and more significant in practical applications.

The measurement of the vertical component $(F_b)$ of the resultant force is obtained by measuring the current I which flows to ground in the schematic circuit diagram of Figure 5, where the generator which supplies the voltage $V_f$ is, preferably, different from the voltage generator to which (in a preferred embodiment of the invention) is connected a resistance divider for supplying each row, respectively each column of the matrix with a voltage proportional to the co-ordinate $(x)$ of the row, respectively to the co-ordinate $(y)$ of the column of the matrix.

In fact, if the conductance of the i-th row of sensors is indicated by $g_i$, we have

$$I = V_f \cdot \sum_{i=1}^{n} g_i = V_f \cdot \beta \cdot \sum_{i=1}^{n} F_i$$

The vertical component $F_b$ of the resultant force is thus correlated to the intensity of the current supplied by the voltage generator $V_f$ connected to one end of the rows of the matrix (which are connected in parallel together), when the other ends of the rows of the matrix are connected to ground. In particular, if the conductance of the sensors is linearly proportional to the pressure, and hence to the vertical component of the force acting on each sensor, the vertical component $F_b$ of the resultant force is proportional to the intensity of the current supplied by the voltage generator $V_f$.

Obviously, the principle continues to hold good if the rows are exchanged with the columns.

Preferably, but not necessarily, the vertical component $F_b$ of the resultant force and the co-ordinates ($X_b$, $Y_b$) of its point of application determined via the schematic circuit diagrams of Figures 3 and 5 are further corrected to take into account the pre-loading forces.

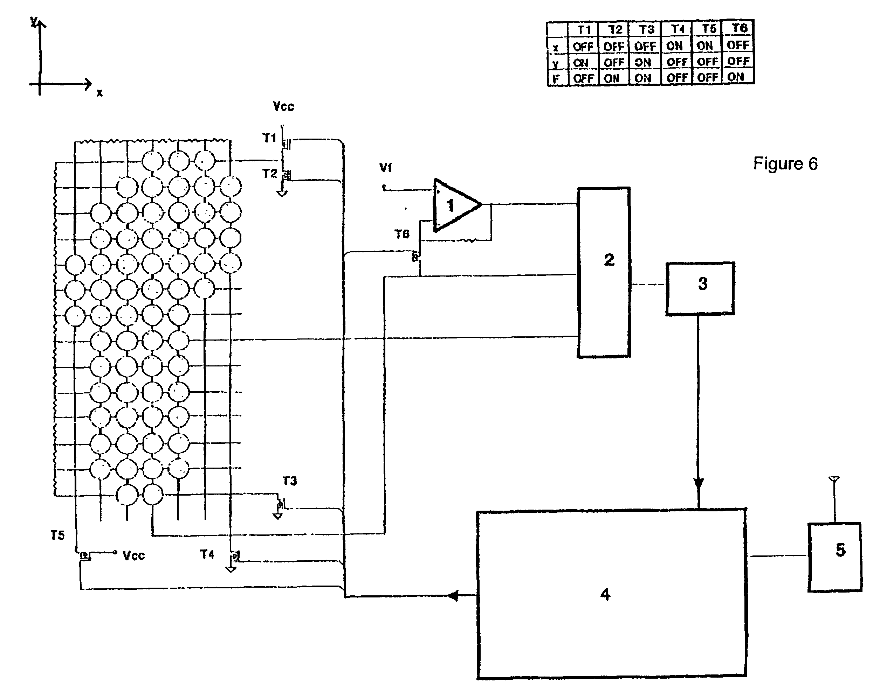

Figure 6 shows a schematic representation of the equivalent circuit of a sensitive insole S (represented by the corresponding matrix of sensors) and the block diagram of the pre-processing unit P associated to it, which comprises at least a logical unit 4 and switching means T (represented in Figure 6 by the MOS transistors T1-T6, which act as switches) activated by the logical unit 4 and suitable for connecting the voltage generators $V_{cc}$ (not explicitly shown in Figure 6) to one end of the resistance divider associated to the rows, respectively to the columns of the matrix, for connecting the voltage generator $V_f$ (not explicitly shown in Figure 6) to one end of the rows, respectively of the columns of the matrix, and for connecting the other ends of the rows and of the columns to the scanning unit 2 (connected to the logical unit 4 via the analog-to-digital converter 3), respectively to ground.

The circuits (schematically shown by the schematic circuit diagrams of Figures 3 and 5) used to measure the co-ordinates ($x_b$, $y_b$) of the centre of application of the resultant force, respectively the vertical component ($F_b$) of the force itself, are realised one at a time by the logical unit 4 (in a cyclical sequence whose frequency is corresponding to the wanted sampling frequency) by activating the switching means T (the MOS transistors T1-T6) according to the "activation matrix" shown in Figure 6 and loaded in tabular form into the memory of the logical unit 4. The logical unit 4 moreover acquires, through the scanning unit 2 and the analog-to-digital converter 3, the signals representing the co-ordinates ($x_b$, $y_b$) of the centre of application of the resultant force, respectively the vertical component ($F_b$) of the force itself, and sends them in serial form to the transmitter T.

The operational amplifier 1, connected in inverting configuration, measures and converts into a voltage the current I which flows to ground, and supplies the voltage $V_f$, in accordance with the schematic circuit diagram of Figure 5.

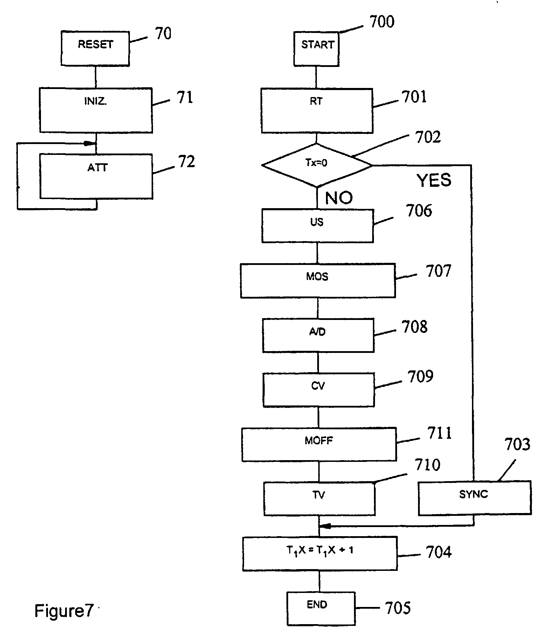

The operational amplifier 1, the scanning unit 2 and the analog-to-digital converter 3 will not be further described because they are known. The working of the logical unit 4 will be better described with reference to the flowchart of Figure 7.

Preferably, the pre-processing unit P is held in a self-supplied container, built in such a way that it can be worn by the subject, and teletransmits via radio the pre-processed digital data to the processing unit PC.

Figure 7 shows a simplified flowchart which better illustrates the working of a logical unit 4, which carries out in order at least the following functional steps:

- when switched on or restarted (RESET; phase 70), after a preset and preliminary checking phase (INIZ; phase 71) the logical unit 4 enters a wait cycle (ATT; 72) which is periodically interrupted by the $Int_0$ routine (phases 700-711), activated (phase 700) by a timer (not explicitly shown because it is known) programmed to generate interrupts with a preset frequency F;

- after activation of the $Int_0$ routine (phase 700), the logical unit 4 resets the timer (RT; phase 701) before verifying (phase 702) the state of a counter ($T_{xl}$), which can assume 4 states, where the 0-state is associated to the transmission of a start-of-cycle synchronism character (SYNC) and the states 1-3 are associated to the acquisition from the sensitive insole S of the vertical component of the resultant force and of the co-ordinates of its point of application;

- if the counter $T_{xl}$ is in the 0-state, the logical unit 4 causes the transmission of the start-of-cycle synchronism character (SYNC; phase 703) before incrementing by one step the counter $T_{xl}$ (phase 704) and ending the $Int_0$ routine (END; phase 705);

- if the counter $T_{xl}$ is in a state other than the 0-state, the logical unit 4 drives the scanning unit 2 (US; phase 706) to acquire from the sensitive insole S the information (the vertical component of the resultant force or one of the co-ordinates of its point of application) associated to the state of the counter $T_{xl}$ ; activates (MOS; phase 707), according to the "activation matrix" shown in Figure 6 and loaded in tabular form into its memory, the MOS transistors T1-T6 which make up the switching means T and activates (A/D; phase 708) the analog-to-digital converter 3.

In a preferred embodiment of the pre-processing unit P, the frequency F is equal to $7 \cdot F_1$ (where $F_1$ is the total sampling frequency for a system including one sensitive insole S) and the logical unit 4 carries out the further functional steps of:

- carry out (CV, phase 709) at least the correction due to the pre-loading force on the digital datum present on the output of the analog-to-digital converter 3;
- modify the digital datum present on the output of the analog-to-digital converter 3 if its digital value corresponds to the start-of-cycle synchronism character (SYNC);
- after the above correction phase (CV; phase 709), deactivate (MOFF; phase 711) the switching means T to reduce the power consumption of the pre-processing unit P;
- start (TD; phase 710) the teletransmission of the pre-processed digital data to the processing unit PC (if present) via the transmitter T;
- activate an universal asynchronous receiver-transmitter (UART), not described herein because it is known, which automatically completes the teletransmission of the pre-processed digital data to the processing unit PC (if present) via the transmitter T during the wait cycle (ATT; 72) of the logical unit 4.

To allow the study of the time relations between the applications of the soles of the two foots on the ground, an apparatus according to the invention may comprise two sensitive insoles S whose responses are acquired by a single pre-processing unit P: said apparatus differs from the previously described one by the fact that:

- the timer generates the interrupts, activating the $Int_0$ routine, with a frequency F equal to 7 • $F_2$, where $F_2$ is the total sampling frequency for a system including two insoles;
- the counter $T_{xl}$ can assume 7 states, where the 0-state is associated to the transmission of the start-of-cycle synchronism character (SYNC), states 1-3 are associated to the acquisition of the vertical component of the resultant force and of the co-ordinates of its point of application from a sensitive insole S, and states 4-7 are associated to their acquisition from the other sensitive insole S;
- the logical unit 4 drives in order the switching means T relative to one, respectively to the other sensitive insole S and drives the scanning unit 2 to acquire in order the vertical component of the resultant force and the co-ordinates of its point of application from one, respectively from the other sensitive insole S.

Also in the embodiment of an apparatus according to the invention suitable for managing two sensitive insoles S, the logical unit 4 carries out the correction phase (CV; phase 709), modifies (if necessary) the digital datum present on the output of the analog-to-digital converter 3, starts (TD; phase 710) the teletransmission of the pre-processed digital data to the processing unit PC (if present) via the transmitter T and activates the universal asynchronous receiver-transmitter (UART).

In a possible embodiment of the invention, the sensitive insole S and at least a part of the pre-processing unit P are built into a shoe to create a measuring apparatus which is practically imperceptible by the foot of the subject.

Without departing from the scope of the invention, it is possible for a skilled person to make to the apparatus object of the present invention all modifications and improvements suggested by the ordinary experience and by the natural evolution of techniques.

**Claims**

1. Apparatus for measuring the vertical component ($F_b$) of the resultant force of the foot-ground interaction and of the co-ordinates ($x_b$, $y_b$) of the point of application of the vertical component ($F_b$) of the resultant force in a reference system integral with the foot, said apparatus comprising at least one sensitive insole (S), to be inserted in a shoe, equipped with a number of force sensors of resistive type, and a pre-processing unit (P) associated to the at least one sensitive insole (S); wherein the force sensors of resistive type belonging to the sensitive insole (S) are arranged in a matrix addressable by rows and columns; wherein one end of each row of the matrix is connected to a generator; wherein one end of each column of the matrix is connected to a generator; wherein the vertical component ($F_b$) of the resultant force is correlated to the intensity of the current supplied by a voltage generator ($V_f$) connected to one end of the rows (of the columns) of the matrix, connected together in parallel, when the other ends of the rows (of the columns) of the matrix are connected to ground; and wherein the pre-processing unit (P) comprises at least one logical unit (4) and switching means (T) activated by the logical unit (4) and suitable for connecting the aforesaid voltage generators to one end of the rows, respectively of the columns of the matrix and for connecting the other ends of the rows, respectively of the columns of the matrix to a scanning unit (2), in turn connected to the logical unit (4) via an analog-to-digital converter (3), respectively to ground;
**characterised in that:**

the generator connected to one end of each row of the matrix can supply a voltage whose intensity is proportional to the co-ordinate ($x$) of said row in the aforesaid reference system, the co-ordinate ($x_b$,) of the point of application of the vertical component ($F_b$) of the resultant force being correlated to the voltage measured at the other ends of the rows of the matrix connected together in parallel;

the generator connected to one end of each column of the matrix can supply a voltage whose intensity is proportional to the co-ordinate ($y$) of said column in the aforesaid reference system, the co-ordinate ($y_b$,) of the point of application of the vertical component ($F_b$) of the resultant force being correlated to the voltage measured at the other ends of the columns of the matrix connected together in parallel.

2. Apparatus according to Claim 1, **characterised in that** the pre-processing unit (P) can teletransmit the pre-processed digital data to a processing unit (PC).

3. Apparatus according to Claim 1, **characterised in that** the generators supplying a voltage whose intensity is proportional to the co-ordinate ($x$) of the row of the matrix of sensors to which each of them is connected are realised by means of a voltage generator and of a resistance divider connected to the voltage generator.

4. Apparatus according to Claim 1, **characterised in that** the generators supplying a voltage whose intensity is proportional to the co-ordinate ($y$) of the column of the matrix of sensors to which each of them is connected are realised by means of a voltage generator and of a resistance divider connected to the voltage generator.

5. Apparatus according to Claim 1, where the conductance of the force sensors of resistive type is linearly proportional to the pressure, **characterised in that** the co-ordinates ($x_b$, respectively $y_b$) of the point of application of the vertical component ($F_b$) of the resultant force are proportional to the voltage measured at an end of the rows of the matrix connected together in parallel, respectively at the end of the columns of the matrix connected together in parallel, when the other end of each row, respectively of each column of the matrix is connected to a generator supplying a voltage whose intensity is proportional to the co-ordinate (x, respectively y) of the row, respectively of the column of the matrix to which said voltage generator is connected; and **in that** the vertical component ($F_b$) of the resultant force is proportional to the intensity of the current supplied by the voltage generator ($V_f$) connected to one end of the rows (of the columns) of the matrix, connected together in parallel, when the other ends of the rows (of the columns) of the matrix are connected to ground.

6. Apparatus according to Claim 1, **characterised in that** the current supplied by the voltage generator ($V_f$) when the rows (the columns) of the matrix are connected to ground is converted into a voltage by an operational amplifier (1), belonging to the pre-processing unit (P), connected in inverting configuration.

7. Apparatus according to Claim 2, **characterised in that** the pre-processing unit (P) is held in a self-supplied container, built in such a way that it can be worn by the subject, and **in that** the pre-processed digital data can be teletransmitted to the processing unit (PC).

8. Apparatus according to Claim 1, **characterised in that** the vertical component ($F_b$) of the resultant force and the co-ordinates ($x_b$, $y_b$) of its point of application are corrected to take into account the pre-loading force.

9. Apparatus according to Claim 1, **characterised in that** the logical unit (4) belonging to the pre-processing unit (P) carries out in order at least the following functional steps:

- when switched on or restarted, after a preset and preliminary checking phase (INIZ), the logical unit (4) can enter a wait cycle (ATT) which is periodically interrupted by a routine ($Int_0$) activated by a timer generating interrupts with a preset frequency (F);
- after activation of the routine ($Int_0$), the logical unit (4) can reset the timer before verifying the state of a counter ($T_{xl}$), which assumes at least 4 states, where the 0-state is associated to the transmission of a start-of-cycle synchronism character (SYNC) and the other states are associated to the acquisition of the vertical component ($F_b$) of the resultant force and of the co-ordinates ($x_b$, $y_b$) of its point of application from the at least one sensitive insole (S);
- if the counter ($T_{xl}$) is in the 0-state, the logical unit (4) can cause the transmission of the start-of-cycle synchronism character (SYNC) before incrementing by one step the counter ($T_{xl}$) and ending the routine ($Int_0$);
- if the counter ($T_{xl}$) is in a state other than the 0-state, the logical unit (4) can drive the scanning unit (2) to acquire from the at least one sensitive insole (S) the information associated to the state of the counter ($T_{xl}$), can activate the switching means (T) and can activate the analog-to-digital converter (3).

**10.** Apparatus according to Claims 8 and 9, **characterised in that** the logical unit (4) can further carry out the correction due to the pre-loading force on the digital datum present on the output of the analog-to-digital converter (3).

**11.** Apparatus according to Claim 9, **characterised in that** the logical unit (4) can further modify the digital datum present on the output of the analog-to-digital converter (3) if the digital value of said digital datum corresponds to the start-of-cycle synchronism character (SYNC).

**12.** Apparatus according to Claim 10 or to Claim 11, **characterised in that**, after the correction of the digital datum present on the output of the analog-to-digital converter (3), the logical unit (4) can deactivate the switching means (T).

**13.** Apparatus according to Claims 2 and 9, **characterised in that**, after activating the analog-to-digital converter (3), the logical unit (4) can start the teletransmission of the pre-processed digital data to the processing unit (PC).

**14.** Apparatus according to Claim 13, **characterised in that** the logical unit (4) can further activate an universal asynchronous receiver-transmitter (UART) which can complete the teletransmission of the pre-processed digital data to the processing unit (PC) during the wait cycle (ATT) of the logical unit (4).

**15.** Apparatus according to Claim 9, **characterised in that** the preset frequency (F) is equal to $7 \cdot F_1$, where $F_1$ is the total sampling frequency for a system including one sensitive insole (S).

**16.** Apparatus according to Claim 1, **characterised in that** the vertical component of the resultant force ($F_b$) and the co-ordinates ($x_b$, $y_b$) of its point of application are acquired from two sensitive insoles (S), one for each foot.

**17.** Apparatus according to Claim 16, **characterised in that** the vertical component ($F_b$) of the resultant force and of the co-ordinates ($x_b$, $y_b$) of its point of application are acquired from both sensitive soles (S) by a single pre-processing unit (P).

**18.** Apparatus according to Claims 9 and 17, **characterised in that**:

- the timer can generate the interruptions, activating the routine ($Int_0$), with a frequency (F) equal to $7 \cdot F_2$, where $F_2$ is the total sampling frequency for a system including two sensitive insoles (S);
- the counter ($T_{xl}$) can assume 7 states, where the 0-state is associated to the transmission of the start-of-cycle synchronism character (SYNC), the states 1-3 are associated to the acquisition of the vertical component ($F_b$) of the resultant force and of the co-ordinates ($x_b$, $y_b$) of its point of application from a sensitive insole (S), and the states 4-7 are associated to the acquisition of the vertical component ($F_b$) of the resultant force and of the co-ordinates ($x_b$, $y_b$) of its point of application from the other sensitive insole (S);
- the logical unit (4) can drive in order the switching means (T) relative to one, respectively to the other sensitive insole (S) and can drive the scanning unit (2) to acquire in order the vertical component ($F_b$) of the resultant force and the co-ordinates ($x_b$, $y_b$) of its point of application from one, respectively from the other sensitive insole (S).

**19.** Apparatus according to Claims 8 and 18, **characterised in that** the logical unit (4) can further carry out the correction due to the pre-loading force on the digital datum present on the output of the analog-to-digital converter (3).

**20.** Apparatus according to Claim 19, **characterised in that** the logical unit (4) can further modify the digital datum present on the output of the analog-to-digital converter (3) if the digital value of said digital datum corresponds the start-of-cycle synchronism character (SYNC).

**21.** Apparatus according to Claim 19 or Claim 20, **characterised in that**, after the correction of the digital datum present on the output of the analog-to-digital converter (3), the logical unit (4) can deactivate the switching means (T).

**22.** Apparatus according to Claim 2 and 18, **characterised in that**, after activating the analog-to-digital converter (3), the logical unit (4) can start the teletransmission of the pre-processed digital data to the processing unit (PC).

**23.** Apparatus according to Claim 22, **characterised in that** the logical unit (4) can activate an universal asynchronous receiver-transmitter (UART) which can complete the teletransmission of the pre-processed digital data to the processing unit (PC) during the wait cycle (ATT) of the logical unit (4).

24. Apparatus according to Claim 1, **characterised in that** the sensitive insole (S) and at least part of the pre-processing unit (P) are built into the shoe.

**Patentansprüche**

1. Vorrichtung zum Messen der vertikalen Komponente ($F_b$) der resultierenden Kraft der Fuß-Boden-Wechselwirkung und der Koordinaten ($x_b'$, $y_b$) des Angriffspunktes der vertikalen Komponente ($F_b$) der resultierenden Kraft in einem Bezugssystem integral mit dem Fuß, wobei die Vorrichtung umfasst: mindestens eine sensitive Einlegesohle (S), die in einen Schuh einzusetzen ist und mit einer Anzahl von Kraftsensoren vom Widerstandstyp ausgestattet ist, und eine Vorverarbeitungseinheit (P), die der mindestens einen sensitiven Einlegesohle (S) zugeordnet ist; wobei die Kraftsensoren vom Widerstandstyp, die zu der sensitiven Einlegesohle (S) gehören, in einer Matrix angeordnet sind, die durch Zeilen und Spalten adressierbar ist,
   wobei ein Ende jeder Zeile der Matrix mit einem Generator verbunden ist;
   wobei ein Ende jeder Spalte der Matrix mit einem Generator verbunden ist;
   wobei die vertikale Komponente ($F_b$) der resultierenden Kraft korreliert ist mit der Intensität des Stromes, der von einem Spannungsgenerator ($V_f$) zugeführt wird, der mit einem Ende der parallel geschalteten Zeilen (Spalten) der Matrix verbunden ist, wenn die anderen Enden der Zeilen (der Spalten) der Matrix mit Masse verbunden sind;
   und wobei die Vorverarbeitungseinheit (P) zumindest eine logische Einheit (4) und ein Schaltmittel (T) umfasst, das durch die logische Einheit (4) aktiviert wird und dafür geeignet ist, die vorstehend erwähnten Spannungsgeneratoren mit einem Ende der Zeilen bzw. der Spalten der Matrix zu verbinden, und die anderen Enden der Zeilen bzw. der Spalten der Matrix mit einer Abtasteinheit (2), die wiederum über einen Analog/ Digital-Wandler (3) mit der logischen Einheit (4) verbunden ist, bzw. mit Masse zu verbinden;
   **dadurch gekennzeichnet, dass**:

   der Generator, der mit einem Ende jeder Zeile der Matrix verbunden ist, eine Spannung zuführen kann, deren Intensität proportional zu der Koordinate (x) der Zeile in dem vorstehend erwähnten Bezugssystem ist, wobei die Koordinate ($x_b'$) des Angriffspunktes der vertikalen Komponente ($F_b$) der resultierenden Kraft mit der Spannung korreliert ist, die an den anderen Enden der parallel geschalteten Zeilen der Matrix gemessen wird;
   der Generator, der mit einem Ende jeder Spalte der Matrix verbunden ist, eine Spannung zuführen kann, deren Intensität proportional zu der Koordinate (y) der Spalte in dem vorstehend erwähnten Bezugssystem ist, wobei die Koordinate ($y_b'$) des Angriffspunktes der vertikalen Komponente ($F_b$) der resultierenden Kraft mit der Spannung korreliert ist, die an den anderen Enden der parallel geschalteten Spalten der Matrix gemessen wird.

2. Vorrichtung nach Anspruch 1, **dadurch gekennzeichnet, dass** die Vorverarbeitungseinheit (P) die vorverarbeiteten digitalen Daten an eine Verarbeitungseinheit (PC) fernübertragen kann.

3. Vorrichtung nach Anspruch 1, **dadurch gekennzeichnet, dass** die Generatoren, die eine Spannung zuführen, deren Intensität proportional zu der Koordinate (x) der Zeile der Matrix von Sensoren ist, mit denen jeder von diesen verbunden ist, mittels eines Spannungsgenerators und eines mit dem Spannungsgenerator verbundenen Widerstandsteilers realisiert sind.

4. Vorrichtung nach Anspruch 1, **dadurch gekennzeichnet, dass** die Generatoren, die eine Spannung zuführen, deren Intensität proportional zu der Koordinate (y) der Spalte der Matrix von Sensoren ist, mit denen jeder von diesen verbunden ist, mittels eines Spannungsgenerators und eines mit dem Spannungsgenerator verbundenen Widerstandsteilers realisiert sind.

5. Vorrichtung nach Anspruch 1, wobei die Leitfähigkeit der Kraftsensoren vom Widerstandstyp linear proportional zu dem Druck ist,
   **dadurch gekennzeichnet, dass** die Koordinaten ($x_b$ bzw. $y_b$) des Angriffspunktes der vertikalen Komponente ($F_b$) der resultierenden Kraft proportional zu der Spannung sind, die an einem Ende der parallel geschalteten Reihen der Matrix bzw. an dem Ende der parallel geschalteten Spalten der Matrix gemessen wird, wenn das andere Ende jeder Reihe bzw. jeder Spalte der Matrix mit einem Generator verbunden ist, der eine Spannung zuführt, deren Intensität proportional zu der Koordinate (x bzw. y) der Reihe bzw. der Spalte der Matrix ist, mit der der Spannungsgenerator verbunden ist; und dass die vertikale Komponente ($F_b$) der resultierenden Kraft proportional zu der Intensität des Stromes ist, der von dem Spannungsgenerator ($V_f$) zugeführt wird, der mit einem Ende der parallel geschalteten Zeilen (Spalten) der Matrix zugeführt wird, wenn die anderen Enden der Zeilen (der Spalten) der Matrix mit Masse verbunden sind.

6. Vorrichtung nach Anspruch 1, **dadurch gekennzeichnet, dass** der Strom, der von dem Spannungsgenerator ($V_f$) zugeführt wird, wenn die Zeilen (die Spalten) der Matrix mit Masse verbunden sind, durch einen Operationsverstärker (1), der zu der Vorverarbeitungseinheit (P) gehört und in eine invertierende Konfiguration geschaltet ist, in eine Spannung umgewandelt wird.

7. Vorrichtung nach Anspruch 2, **dadurch gekennzeichnet, dass** die Vorverarbeitungseinheit (P) in einem eigenversorgten Behälter gehalten ist, der auf eine solche Weise aufgebaut ist, dass er von der Person getragen werden kann, und dass die vorverarbeiteten digitalen Daten an die Verarbeitungseinheit (PC) fernübertragen werden können.

8. Vorrichtung nach Anspruch 1, **dadurch gekennzeichnet, dass** die vertikale Komponente ($F_b$) der resultierenden Kraft und die Koordinaten ($x_b$, $y_b$) ihres Angriffspunktes korrigiert werden, um die Vorbelastungskraft zu berücksichtigen.

9. Vorrichtung nach Anspruch 1, **dadurch gekennzeichnet, dass** die logische Einheit (4), die zu der Vorverarbeitungseinheit (P) gehört, in der angegebenen Reihenfolge zumindest die folgenden funktionalen Schritte ausführt:

   - beim Einschalten oder Neustarten nach einer Voreinstellungsund vorläufigen Prüfphase (INIZ) die logische Einheit (4) in einen Wartezyklus (ATT) eintreten kann, der von einer Routine ($Int_0$) periodisch unterbrochen wird, die von einem Zeitglied aktiviert wird, das Unterbrechungen mit einer voreingestellten Frequenz (F) erzeugt;
   - nach der Aktivierung der Routine ($Int_0$) die logische Einheit (4) das Zeitglied zurücksetzen kann, bevor der Zustand eines Zählers ($T_{xl}$) überprüft wird, der zumindest vier Zustände annimmt, wobei der 0-Zustand zu der Übertragung eines Start-von-Zyklus-Synchronzeichens (SYNC) gehört, und die anderen Zustände zu der Beschaffung der vertikalen Komponente ($F_b$) der resultierenden Kraft und der Koordinaten ($x_b$, $y_b$) ihres Angriffspunktes von der zumindest einen sensitiven Einlegesohle (S) gehören;
   - bei im 0-Zustand befindlichem Zähler ($T_{xl}$) die logische Einheit (4) die Übertragung des Start-von-Zyklus-Synchronzeichens (SYNC) bewirken kann, bevor der Zähler ($T_{xl}$) um einen Schritt inkrementiert wird und die Routine ($Int_0$) beendet wird;
   - bei in einem anderen Zustand als dem 0-Zustand befindlichem Zähler ($T_{xl}$) die logische Einheit (4) die Abtasteinheit (2) ansteuern kann, um von der zumindest einen sensitiven Einlegesohle (S) die Information zu beschaffen, die zu dem Zustand des Zählers ($T_{xl}$) gehört, das Schaltmittel (T) aktivieren kann und den Analog/ Digital-Wandler (3) aktivieren kann.

10. Vorrichtung nach den Ansprüchen 8 und 9, **dadurch gekennzeichnet, dass** die logische Einheit (4) ferner die Korrektur in Bezug auf die Vorbelastungskraft an dem digitalen Datenelement, das am Ausgang des Analog/ Digital-Wandlers (3) vorhanden ist, ausführen kann.

11. Vorrichtung nach Anspruch 9, **dadurch gekennzeichnet, dass** die logische Einheit (4) ferner das digitale Datenelement, das am Ausgang des Analog/Digital-Wandlers (3) vorhanden ist, modifizieren kann, wenn der digitale Wert des digitalen Datenelements dem Start-von-Zyklus-Synchronzeichen (SYNC) entspricht.

12. Vorrichtung nach Anspruch 10 oder Anspruch 11, **dadurch gekennzeichnet, dass** nach der Korrektur des digitalen Datenelements, das am Ausgang des Analog/ Digital-Wandlers (3) vorhanden ist, die logische Einheit (4) das Schaltmittel (T) deaktivieren kann.

13. Vorrichtung nach Anspruch 2 und 9, **dadurch gekennzeichnet, dass** nach dem Aktivieren des Analog/Digital-Wandlers (3) die logische Einheit (4) die Fernübertragung der vorverarbeiteten digitalen Daten an die Verarbeitungseinheit (PC) starten kann.

14. Vorrichtung nach Anspruch 13, **dadurch gekennzeichnet, dass** die logische Einheit (4) ferner einen universalen asynchronen Sender-Empfänger (UART) aktivieren kann, der die Fernübertragung der vorverarbeiteten digitalen Daten an die Verarbeitungseinheit (PC) während des Wartezyklus (ATT) der logischen Einheit (4) abschließen kann.

**15.** Vorrichtung nach Anspruch 9, **dadurch gekennzeichnet, dass** die voreingestellte Frequenz (F) gleich $7 \cdot F_1$ ist, wobei $F_1$ die Gesamtabtastfrequenz für ein System ist, das eine sensitive Einlegesohle (S) umfasst.

**16.** Vorrichtung nach Anspruch 1, **dadurch gekennzeichnet, dass** die vertikale Komponente der resultierenden Kraft ($F_b$) und die Koordinaten ($x_b$, $y_b$) ihres Angriffspunktes von zwei sensitiven Einlegesohlen (S), und zwar eine für jeden Fuß, beschafft werden.

**17.** Vorrichtung nach Anspruch 16, **dadurch gekennzeichnet, dass** die vertikale Komponente ($F_b$) der resultierenden Kraft und die Koordinaten ($x_b$, $y_b$) ihres Angriffspunktes von beiden sensitiven Einlegesohlen (S) durch eine einzige Vorverarbeitungseinheit (P) beschafft werden.

**18.** Vorrichtung nach Anspruch 9 und 17, **dadurch gekennzeichnet, dass**:

- das Zeitglied die Unterbrechungen erzeugen kann, die Routine (Into) mit einer Frequenz (F) gleich $7 \cdot F_2$ aktivieren kann, wobei $F_2$ die Gesamtabtastfrequenz für ein System ist, das zwei sensitive Einlegesohlen (S) umfasst;
- der Zähler ($T_{xl}$) 7 Zustände annehmen kann, wobei der 0-Zustand zu der Übertragung des Start-von-Zyklus-Synchronzeichens (SYNC) gehört, die Zustände 1-3 zu der Beschaffung der vertikalen Komponente ($F_b$) der resultierenden Kraft und der Koordinaten ($x_b$, $y_b$) ihres Angriffspunktes von einer sensitiven Einlegesohle (S) gehören, und die Zustände 4-7 zu der Beschaffung der vertikalen Komponente ($F_b$) der resultierenden Kraft und der Koordinaten ($x_b$, $y_b$) ihres Angriffspunktes von der anderen sensitiven Einlegesohle (S) gehören;
- die logische Einheit (4) in der Reihenfolge das Schaltmittel (T) in Bezug auf die eine bzw. auf die andere sensitive Einlegesohle (S) ansteuern kann und die Abtasteinheit (2) ansteuern kann, um in der Reihenfolge die vertikale Komponente ($F_b$) der resultierenden Kraft und die Koordinaten ($x_b$, $y_b$) Ihres Angriffspunktes von der einen bzw. von der anderen sensitiven Einlegesohle (S) zu beschaffen.

**19.** Vorrichtung nach Anspruch 8 und 18, **dadurch gekennzeichnet, dass** die logische Einheit (4) ferner die Korrektur in Bezug auf die Vorbelastungskraft auf das digitale Datenelement, das am Ausgang des Analog/Digital-Wandlers (3) vorhanden ist, ausführen kann.

**20.** Vorrichtung nach Anspruch 19, **dadurch gekennzeichnet, dass** die logische Einheit (4) ferner das digitale Datenelement, das am Ausgang des Analog/Digital-Wandlers (3) vorhanden ist, modifizieren kann, wenn der digitale Wert des digitalen Datenelements dem Start-von-Zyklus-Synchronzeichen (SYNC) entspricht.

**21.** Vorrichtung nach Anspruch 19 oder Anspruch 20, **dadurch gekennzeichnet, dass** nach der Korrektur des digitalen Datenelements, das am Ausgang des Analog/Digital-Wandlers (3) vorhanden ist, die logische Einheit (4) das Schaltmittel (T) deaktivieren kann.

**22.** Vorrichtung nach Anspruch 2 und 18, **dadurch gekennzeichnet, dass** nach dem Aktivieren des Analog/ Digital-Wandlers (3) die logische Einheit (4) die Fernübertragung der vorverarbeiteten digitalen Daten an die Verarbeitungseinheit (PC) starten kann.

**23.** Vorrichtung nach Anspruch 22, **dadurch gekennzeichnet, dass** die logische Einheit (4) einen universellen asynchronen Sender-Empfänger (UART) aktivieren kann, der die Fernübertragung der vorverarbeiteten digitalen Daten an die Verarbeitungseinheit (PC) während des Wartezyklus (ATT) der logischen Einheit (4) abschließen kann.

**24.** Vorrichtung nach Anspruch 1, **dadurch gekennzeichnet, dass** die sensitive Einlegesohle (S) und zumindest ein Teil der Vorverarbeitungseinheit (P) in den Schuh eingebaut sind.

**Revendications**

**1.** Appareil de mesure de la composante verticale ($F_b$) de la force résultant d'une interaction entre le pied et le sol et l'une des coordonnées ($x_{b'}$, $y_b$) du point d'application de la composante verticale ($F_b$) de la force résultante dans un système de référence intégral avec le pied, ledit appareil comprenant au moins une première semelle sensible (S), à insérer dans une chaussure, équipée d'un nombre de capteurs de force du type résistif, et une unité de pré-traitement (P) associée à au moins une première semelle sensible (S); où les capteurs de force du type résistif appartenant à la première semelle sensible (S) sont agencés selon une matrice pouvant être adressée par des rangées et des colonnes; où une extrémité de chaque rangée de la matrice est reliée à un générateur; où une extrémité de chaque colonne de la matrice est reliée à un générateur; où le composant vertical ($F_b$) de la force résultante est en corrélation avec l'intensité du courant fourni par un générateur de tension ($V_f$) connecté à une extrémité des rangées (des colonnes) de la matrice, connectées ensemble en parallèle, lorsque les autres extrémités des rangées (des colonnes) de la matrice sont reliées à la masse.;

et où l'unité de pré-traitement (P) comprend au moins une unité logique (4) et un moyen de commutation (T) activé par l'unité logique (4) et apte à relier les générateurs de tension précités à une extrémité des rangées, respectivement des colonnes de la matrice et pour relier les autres extrémités des rangées, respectivement des colonnes de la matrice à une unité de balayage (2), reliée à son tour à l'unité logique (4) par un convertisseur analogique/numérique (3), respectivement à la masse; **caractérisé en ce que**:

le générateur relié à une extrémité de chaque rangée de la matrice peut fournir une tension dont l'intensité est proportionnelle à la coordonnée (x) de la rangée dans le système de référence précité, la coordonnée ($x_{b'}$) du point d'application de la composante verticale ($F_b$) de la force résultante étant en corrélation avec la tension mesurée aux autres extrémités des rangées de la matrice reliées ensemble parallèlement;

le générateur relié à une extrémité de chaque colonne de la matrice peut fournir une tension dont l'intensité est proportionnelle à la coordonnée (y) de ladite colonne dans ledit système de référence, la coordonnée ($y_{b'}$) du point d'application de la composante verticale ($F_b$) dé la force résultante étant en corrélation avec la tension mesurée aux autres extrémités des colonnes de la matrice reliées ensemble parallèlement.

2. Appareil selon la revendication 1, **caractérisé en ce que** l'unité de pré-traitement (P) peut télétransmettre les données numériques pré-traitées à une unité de traitement (PC).

3. Appareil selon la revendication 1, **caractérisé en ce que** les générateurs fournissant une tension dont l'intensité est proportionnelle à la coordonnée (x) de la rangée de la matrice de capteurs à laquelle chacun d'eux est relié, sont réalisés au moyen d'un générateur de tension et d'un diviseur de résistance relié au générateur de tension.

4. Appareil selon la revendication 1, **caractérisé en ce que** les générateurs fournissant une tension dont l'intensité est proportionnelle à la coordonnée (y) de la colonne de la matrice de capteurs à laquelle chacun d'eux est relié, sont réalisés au moyen d'un générateur de tension et d'un diviseur de résistance relié au générateur de tension.

5. Appareil selon la revendication 1, où la conductance des capteurs de force du type résistif est linéairement proportionnelle à la pression, **caractérisé en ce que** les coordonnées ($x_b$, respectivement $y_b$) du point d'application de la composante verticale ($F_b$) de la force résultante sont proportionnelles à la tension mesurée à une extrémité des rangées de la matrice reliées ensemble parallèlement, respectivement à l'extrémité des colonnes de la matrice reliées ensemble parallèlement, lorsque l'autre extrémité de chaque rangée, respectivement, de chaque colonne de la matrice, est reliée à un générateur fournissant une tension dont l'intensité est proportionnelle à la coordonnée (x, respectivement y) de la rangée, respectivement de la colonne de la matrice à laquelle ledit générateur de tension est relié; et **en ce que** la composante verticale ($F_b$) de la force résultante est proportionnelle à l'intensité du courant fourni par le générateur de tension ($V_f$) relié à une extrémité des rangées (des colonnes) de la matrice, reliées ensemble parallèlement, lorsque les autres extrémités des rangées (des colonnes) de la matrice sont reliées à la masse.

6. Appareil selon la revendication 1, **caractérisé en ce que** le courant fourni par le générateur de tension ($V_f$), lorsque les rangées (les colonnes) de la matrice sont reliées à la masse, est converti en une tension par un amplificateur opérationnel (1) faisant partie de l'unité de pré-traitement (P), reliées selon une configuration inversée.

7. Appareil selon la revendication 2, **caractérisé en ce que** l'unité de pré-traitement (P) est retenue dans un boîtier auto-alimenté, construit de telle manière qu'il peut être porté par le sujet, et **en ce que** les données numériques pré-traitées peuvent être télé-transmises à l'unité de traitement (PC).

8. Appareil selon la revendication 1, **caractérisé en ce que** la composante verticale ($F_b$) de la force résultante et les coordonnées ($x_b$, $y_b$) de son point d'application sont corrigées pour tenir compte de la force de pré-chargement.

9. Appareil selon la revendication 1, **caractérisé en ce que** l'unité logique (4) faisant partie de l'unité de pré-traitement (P) exécute dans l'ordre au moins les étapes fonctionnelles suivantes :

- lorsqu'elle est mise en service ou redémarrée, après une phase de vérification pré-établie et préliminaire (INIZ), l'unité logique (4) peut entrer dans un cycle d'attente (ATT) qui est périodiquement interrompu par une routine ($Int_o$) activée par une horloge générant des interruptions selon une fréquence préréglée (F);
- après l'activation de la routine ($Int_o$), l'unité logique (4) peut remettre à l'état initial l'horloge avant de vérifier l'état d'un compteur ($T_{x1}$), qui présente au moins 4 états, où l'état-0 est associé à la transmission d'un caractère de synchronisation de démarrage de cycle (SYNC) et les autres états sont associés à l'acquisition de la composante verticale ($F_b$) de la force résultante et des co-ordonnées ($x_b$, $y_b$) de son point d'application d'au moins une première semelle sensible (S);
- si le compteur ($T_{x1}$) se trouve à l'état-0, l'unité logique (4) peut provoquer la transmission du caractère de synchronisation de démarrage de cycle (SYNC) avant d'incrémenter le compteur d'une étape ($T_{x1}$) et de terminer la routine ($Int_o$);
- si le compteur ($T_{x1}$) se trouve dans un état autre que l'état-0, l'unité logique (4) peut entraîner l'unité de balayage (2) pour acquérir de ladite au moins une première semelle sensible (S) les informations associées à l'état du compteur ($T_{x1}$), peut activer le moyen de commutation (T) et peut activer le convertisseur analogique/numérique (3).

10. Appareil selon les revendications 8 et 9, **caractérisé en ce que** l'unité logique (4) peut exécuter en outre la correction due à la force de pré-charge sur la donnée numérique présente à la sortie du convertisseur analogique/numérique (3).

11. Appareil selon la revendication 9, **caractérisé en ce que** l'unité logique (4) peut modifier en outre la donnée numérique présente à la sortie du convertisseur analogique/numérique (3) si la valeur numérique de ladite donnée numérique correspond au caractère de synchronisation de démarrage de cycle (SYNC).

12. Appareil selon la revendication 10 ou la revendication 11, **caractérisé en ce que**, après la correction de la donnée numérique présente à la sortie du convertisseur analogique/numérique (3), l'unité logique (4) peut désactiver le moyen de commutation (T).

13. Appareil selon les revendications 2 et 9, **caractérisé en ce que**, après l'activation du convertisseur analogique/numérique (3), l'unité logique (4) peut démarrer la télétransmission des données numériques pré-traitées à l'unité de traitement (PC).

14. Appareil selon la revendication 13, **caractérisé en ce que** l'unité logique (4) peut activer en outre un récepteur-transmetteur asynchrone universel (UART) qui peut compléter la télétransmission des données numériques pré-traitées à l'unité de traitement (PC) pendant le cycle d'attente (ATT) de l'unité logique (4).

15. Appareil selon la revendication 9, **caractérisé en ce que** la fréquence pré-réglée (F) est égale à $7.F_1$, où $F_1$ représente la fréquence d'échantillonnage totale d'un système comprenant une première semelle sensible (S).

16. Appareil selon la revendication 1, **caractérisé en ce que** la composante verticale de la force résultante (Fb) et les coordonnées ($x_b$, $y_b$) de son point d'application sont acquises de deux premières semelles sensibles (S), une pour chaque pied.

17. Appareil selon la revendication 16, **caractérisé en ce que** la composante verticale ($F_b$) de la force résultante et les co-ordonnées (xb, yb) de son point d'application sont acquises des deux semelles sensibles (S) par une seule unité de pré-traitement (P).

18. Appareil selon les revendications 9 et 17, **caractérisé en ce que** :

- l'horloge peut produire les interruptions, activer la routine ($Int_o$), avec une fréquence (F) égale à $7 . F_2$, où $F_2$ représente la fréquence d'échantillonnage totale d'un système comprenant deux premières semelles sensibles (S);
- le compteur ($T_{x1}$) peut occuper 7 états, où l'état-0 est associé à la transmission du caractère de synchronisation de démarrage de cycle (SYNC), les états 1-3 sont associés à l'acquisition de la composante verticale ($F_b$) de la force résultante et des coordonnées ($x_b$, $y_b$) de son point d'application de la première semelle sensible (S), et les états 4-7 sont associés à l'acquisition de la composante verticale ($F_b$) de la force résultante et des coordonnées ($x_b$, $y_b$) de son point d'application de l'autre première semelle sensible (S);
- l'unité logique (4) peut entraîner dans l'ordre le moyen de commutation (T) relativement à l'une, respectivement à l'autre première semelle sensible (S) et peut entraîner l'unité de balayage (2) pour acquérir dans l'ordre la composante verticale ($F_b$) de la force résultante et les coordonnées ($x_b$, $y_b$) de son point d'application de l'une, respectivement de l'autre première semelle sensible (S).

19. Appareil selon les revendications 8 et 18, **caractérisé en ce que** l'unité logique (4) peut exécuter en outre la correction due à la force de pré-charge sur la donnée numérique présente à la sortie du convertisseur analogique/ numérique (3).

20. Appareil selon la revendication 19, **caractérisé en ce que** l'unité logique (4) peut modifier en outre la donnée numérique présente à la sortie du convertisseur analogique/numérique (3) si la valeur numérique de ladite donnée numérique correspond au caractère de synchronisation du démarrage de cycle (SYNC).

21. Appareil selon la revendication 19 ou la revendication 20, **caractérisé en ce que**, après la correction de la donnée numérique présente à la sortie du convertisseur analogique/numérique (3), l'unité logique (4) peut désactiver le moyen de commutation (T).

22. Appareil selon les revendications 2 et 18, **caractérisé en ce que**, après l'activation du convertisseur analogique/ numérique (3), l'unité logique (4) peut démarrer la télétransmission des données numériques pré-traitées à l'unité de traitement (PC).

23. Appareil selon la revendication 22, **caractérisé en ce que** l'unité logique (4) peut activer un récepteur/transmetteur asynchrone universel (UART) qui peut compléter la télétransmission des données numériques pré-traitées à l'unité de traitement (PC) pendant le cycle d'attente (ATT) de l'unité logique (4).

24. Appareil selon la revendication 1, **caractérisé en ce que** la première semelle sensible (S) et au moins une partie de l'unité de pré-traitement (P) sont intégrées dans la chaussure.

Figure 1

Figure 2

Figure 3

Figure 4

Figure 5

Figure 6

| | T1 | T2 | T3 | T4 | T5 | T6 |
|---|---|---|---|---|---|---|
| x | OFF | OFF | OFF | ON | ON | OFF |
| y | ON | OFF | ON | OFF | OFF | OFF |
| F | OFF | ON | ON | OFF | OFF | ON |

EP 0 846 441 B1

Figure7